# EUROPEAN PATENT SPECIFICATION

(11) **EP 1 671 592 B1**
(45) Date of publication and mention of the grant of the patent: **25.08.2010**
(21) Application number: 05111386.8
(22) Date of filing: 28.11.2005
(51) Int. Cl.: A61B 17/00

(54) **Medical sealing device**
Medizinische Dichtungsvorrichtung
Système d'étanchéité médical

(30) Priority: 16.12.2004 SE 4030706
(43) Date of publication of application: 21.06.2006
(73) Proprietor: RADI MEDICAL SYSTEMS AB, 754 50 Uppsala (SE)
(72) Inventor: Egnelöv, Per, 754 50, UPPSALA (SE)
(74) Representative: Holmberg, Nils Anders Patrik

(56) References cited:
- WO-A-98/31287
- US-A- 5 662 681
- US-A- 5 683 411
- US-B1- 6 315 787
- US-B1- 6 508 828

## Description

### Field of the invention.

The present invention relates generally to the field of sealing devices for the sealing of a percutaneous puncture in a vessel wall, and in particular to the class of sealing devices that comprises an intra-arterial member and an extra-arterial member, which are sandwiching the vessel wall and are held together by a retaining member, and more particularly to a sealing element which is positioned between the extra-arterial member and the vessel wall to improve the sealing performance of the sealing device.

### Background of the invention

In the U.S. Patent No. 6,508,828, which is assigned to the present assignee, a sealing device is disclosed for sealing a puncture hole in a vessel wall. The sealing device comprises an inner sealing member, an outer member, and a retaining member. The inner sealing member is adapted to be positioned at the inner wall of a vessel, while the outer member is adapted to be positioned at the outer wall of the vessel. In use, the inner and outer members are sandwiching the vessel wall, and are held together by the retaining member to thereby seal the puncture hole in the vessel wall.

In U.S. Patent No. 6,596,012, which also is assigned to the present assignee, it is described how the sealing action of an inner sealing member can be improved by providing the inner sealing member with a rim portion that has a lower structural rigidity than a central portion of the inner sealing member.

Other examples of sealing devices that comprise an inner member and an outer member, which are held together by an elongated retaining member, such as a suture or filament, can be found in, for example, U.S. Patent Nos. 5,593,422 and 5,620,461. In U.S. Patent No. 5,342,393, the retaining member is in the form of a stem that extends from the inner member.

US 6315787 B1 discloses a vessel plug having a handle which include a proximal end and a distal end, a flange attached to the distal end of the handle, and a tip extending distally beyond the flange. In use, the flange and the tip are shaped to engage an opening of a blood vessel, provide occlusion to the opening of the vessel to block the blood flow, thereby achieving hemostasis.

Although at least a sealing device designed according to the teachings of U.S. Patent Nos. 6,508,828 and 6,596,012 in practice has proven to work very well, its sealing function can be improved. The general object of the present invention is therefore to provide a sealing device with an enhanced sealing capacity. Preferably, the invention should be applicable to an existing sealing device without changing the design of the other components of the sealing device, or changing the practical handling of the sealing device.

### Summary of the invention

The above-mentioned objects are achieved with a sealing device according to the independent claim.

Preferred embodiments are set forth in the dependent claims.

The present invention is related to a sealing device comprising an intra-arterial (inner) member and an extra-arterial (outer) member, which are held together by a retaining member. In use, the inner member is through a puncture hole in a vessel wall introduced into the lumen of the vessel, and is then retracted until it is in close contact with the inner vessel wall. The retaining member, which is attached to the inner member, then extends through the puncture hole and holds the inner member tightly in a fixed position. The outer member is then advanced along the retaining member until the outer member is contacting the outside of the vessel wall. When the operation is completed, the outer and inner members will thereby sandwich the vessel wall and the puncture hole therein.

The actual sealing of the puncture hole can in principle be accomplished by two different mechanisms, either by clamping the vessel wall between the inner and outer members, or by the inner member alone. In the latter case, the outer member merely acts as a locking disc, which holds the inner sealing member in place. For the purpose of the present invention, it is rather irrelevant which one of these two effects that actually accomplishes the sealing of the puncture hole. (For the sake of completeness, a sealing device comprising an intra-arterial anchor member and an extra-arterial sealing member, such that the sealing is accomplished outside the vessel wall, is not considered to fall within the scope of the present invention.)

According to the invention, the sealing performance of a sealing device comprising an inner member, an outer member and a retaining member can be improved by providing a sealing element which is adapted to be positioned between the outer member and an outer wall of a vessel. The sealing element will thereby act as a washer or packing that supports the normal sealing action of the outer and inner members.

The sealing element should preferably be made from a soft and pliable material that conforms to the tissue surrounding the vessel. It is further preferred that the material is a bioresorbable material that resorbs in a patient's body. In one embodiment of the present invention, the sealing element exhibits an open porous structure.

In another embodiment of the invention, the sealing element comprises a haemostatic agent which promotes the sealing of a puncture wound in a vessel wall. The haemostatic agent can be provided as an exterior layer, or can be incorporated in the matrix of a porous material.

### Brief description of the drawings

Fig. 1 is a schematic illustration of the components of a sealing device according to the present invention.
Fig. 2 shows the sealing device of Fig. 1 in a state corresponding to the completion of a medical sealing operation.

### Detailed description of the invention

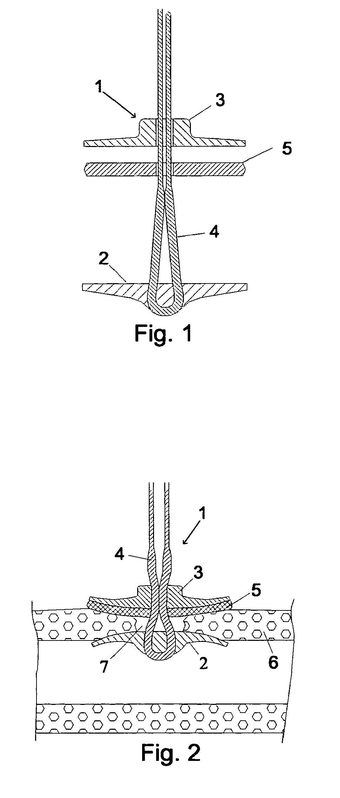

A sealing or closure device 1 according to the present invention is schematically illustrated in Fig. 1. The sealing device 1 comprises an inner member 2 and an outer member 3, which are held together be an elongated retaining member 4. The inner member 2, the outer member 3 and the retaining member 4 are all of previously known designs, examples of which can be found in the above-referenced patents. The retaining member 4 is attached to the inner member 2, and extends through a hole in the outer member 3. During the positioning operation of the sealing device 1, the outer member 3 can thereby slide along the retaining member 4 into abutment against the outer surface of a vessel. Unlike the previously known sealing devices, the sealing device 1 comprises further an extra sealing element 5, which in this example has the form of a relatively thin sealing disc or washer 5.

The function of the sealing disc 5 is clearly shown in Fig. 2, where the sealing device 1 has been positioned around a vessel wall 6 in order to close a puncture hole 7 therein. The figure illustrates that the inner member 2 is positioned at an interior surface of the vessel wall 6, while the outer member 3 is positioned at an exterior surface of the vessel wall 6, and that the retaining member 4 extends through the puncture hole 7 in the vessel wall 6, such that the vessel wall 6 is sandwiched between the inner member 2 and the outer member 3. In this example, a portion of the retaining member 4 has been provided with an enlarged thickness, such that the outer member 3 is held in a fixed position by the friction acting between the retaining member 4 and the outer member 3. It should, however, be understood that other ways of securing an outer member to a retaining member could be provided, for example by having a retaining member in the form of a saw-toothed stem. Although not shown in the figures, it is preferred that the curvatures of the inner and outer members are adapted to the curvature of the vessel wall.

In the example shown in Figs. 1 and 2, a sealing element 5 is provided as a separate part of a sealing device 1.

A sealing element, such as the sealing disc 5 shown in Figs. 1 and 2, is preferably made from a soft and pliable material that conforms to the shape and texture of the vessel wall. The sealing element will thereby act as a packing or seal that mechanically prevents a leakage between an outer member and the vessel wall. Such a leakage could otherwise arise from the forming of small canals between a sealing or closure device and the tissue around a puncture wound. A sealing element according to the present invention is relatively thin and has a thickness that is comparable to the thicknesses of the inner and outer members. The thickness is preferably less than 5 mm, and more preferably 3-4 mm. As an alternative, a sealing element could have a non-uniform thickness, with a larger thickness in the centre of the sealing element than in its periphery. The central portion could then be arranged as a cone-shaped structure that can protrude a short distance into a hole in a vessel wall. Preferably, the diameter of a sealing element is essentially equal to the diameter of an outer member.

To guarantee a high degree of compliance, a sealing element could be made from a material that exhibits an open porous structure. The porosity of the material could be in the range from 50 to 90 percent. By making the sealing element from a porous material, the compliance of the sealing element could be modified by modifying the porosity of the material, in addition to modifying the material composition itself. Preferably, an outer assembly, which consists of an outer member and a sealing element, would then have a continuous transition from a homogenous structure to an open porous structure in the direction towards a vessel wall.

Whether or not the material in the sealing element exhibits a porous structure, the material should preferable be a bioresorbable material. To allow a close adaptation to the surrounding tissue, the material structure should be able to accommodate large deformations without breaking, and should therefore have a glass transition temperature below room temperature. Non-limiting examples of such synthetic resorbable materials are various combinations of the monomers glycolide, lactide (all stereoismers), trimethylene carbonate, ε-caprolactone, dioxanone or dioxepanone. Depending on the desired mechanical properties and the choice of manufacturing method, several of the homopolymers or copolymers containing two or more of the above-mentioned monomers can be used to manufacture a sealing element, including a porous sealing element. Other examples of synthetic resorbable polymers that can be utilized is various aliphatic polyurethanes, such as polyureaurethanes, polyesterurethanes and polycarbonateurethanes, and yet other materials such as polyphosphazenes or polyorthoesters.

The sealing element, including a porous sealing element, can also be made from natural biomaterials. Several resorbable and naturally occurring materials exist that will fulfill the requirements above. Non-limiting examples of suitable natural biomaterials are various forms of collagen, hyaluronic acid, alginic acid, fibrin, starch and hemicelluloses.

An open porous structure can be fabricated with a plurality of different known techniques, including leaching of added components, such as salt, thermally or chemically induced phase separation techniques, and sublimation.

In yet another embodiment of the present invention, a sealing element is supplemented with a haemostatic agent, which promotes the coagulation process. In addition to its mechanical sealing properties, the sealing element then acts as a substrate or carrier material for the haemostatic agent. If the sealing element comprises an open porous structure, the haemostatic agent can be incorporated in the matrix structure. If the sealing element instead is made from a non-porous material, the sealing element can be coated with the haemostatic agent, which then is provided as a thin layer. Suitable haemostatic agents are exemplified by, but not limited to, thrombin, pro-thrombin, coagulation factor XIIa, factor Va, factor Xa, or tranexamic acid.

Although the present invention has been described with reference to specific embodiments, also shown in the appended drawings, it will be apparent for those skilled in the art that many variations and modifications can be done within the scope of the invention as described in the specification and defined with reference to the claims below.

## Claims

1. Medical sealing device (1) for the sealing of a puncture hole in a vessel wall, comprising an inner member (2), which is adapted to be positioned at an interior surface of the vessel wall, and an outer member (3), which is adapted to be positioned outside the vessel wall, the inner member (2) and the other member (3) being held together by a retaining member (4), **characterized in that** the sealing device (1) further comprises a sealing element (5) which is a separate part of the sealing device (1) and which is adapted to be positioned between the vessel wall and the outer member (3), the sealing element is made from a soft and compliable material and the diameter of the sealing element (5) is essentially equal to the diameter of the outer member (3) and has a thickness that is comparable to the thicknesses of the inner and outer members, and that said sealing element (5) is made from a bioresorbable material.

2. Medical sealing device (1) according to claim 1, **characterized in that** the sealing element (5) has a larger thickness in a central portion than in a peripheral portion.

3. Medical sealing device (1) according to claim 2, **characterized in that** said central portion has a cone-shaped structure that can protrude a short distance into said hole in said vessel wall.

4. Medical sealing device (1) according to any of claims 1-3, **characterized in that** the thickness of the sealing element (5) is less than 5 mm.

5. Medical sealing device (1) according to anyone of the previous claims, **characterized in that** the medical sealing device (1) is made from a porous material.

6. Medical sealing device (1) according to claim 5, **characterized in that** the porosity of the material ranges from 50 to 95 percent.

7. Medical sealing device (1) according to anyone of the previous claims, **characterized in that** the sealing element (5) is made from glycolide, lactide, trimethylene carbonate, ε-caprolactone, dioxanone, dioxepanone, polyurethane, polyphosphazenes, polyorthoesters, collagen, hyaluronic acid, alginic acid, fibrin, starch or hemicellulose, or combinations thereof.

8. Medical sealing device (1) according to anyone of the previous claims, **characterized in that** the sealing element further comprises a haemostatic agent.

9. Medical sealing device (1) according to claim 8, **characterized in that** the haemostatic agent is chosen from the group comprising thrombin, pro-thrombin, coagulation factor XIIa, factor Va, factor Xa, or tranexamic acid.

## Patentansprüche

1. Medizinische Dichtungsvorrichtung (1) zum Abdichten eines Punktionslochs in einer Gefäßwand, die ein Innenelement (2), das an einer Innenseite der Gefäßwand angeordnet werden kann, und ein Außenelement (3), das außerhalb der Gefäßwand angeordnet werden kann, umfasst, wobei das Innenelement (2) und das Außenelement (3) von einem Halteelement (4) zusammengehalten werden, **dadurch gekennzeichnet, dass** die Dichtungsvorrichtung (1) ferner ein Abdichtelement (5) umfasst, das ein separater Teil der Dichtungsvorrichtung (1) ist und zwischen der Gefäßwand und dem Außenelement (3) angeordnet werden kann, wobei das Abdichtelement aus einem weichen und nachgiebigen Material besteht und der Durchmesser des abdichtelementes (5) im Wesentlichen dem Durchmesser des Außenelements (3) entspricht und eine Dicke aufweist, die mit den Dicken des Innen- und Außenelements vergleichbar ist, und das das Abdichtelement (5) aus einem bioresorbierbaren Material besteht.

2. Medizinische Dichtungsvorrichtung (1) nach Anspruch 1, **dadurch gekennzeichnet, dass** das Abdichtelement (5) in einem mittleren Teil eine größere Dicke aufweiset als in einem peripheren Teil.

3. Medizinische Dichtungsvorrichtung (1) nach Anspruch 2, **dadurch gekennzeichnet, dass** der mittlere Teil eine konusförmige Gestalt aufweist, die eine kurze Strecke in das Loch in der Gefäßwand hinein ragen kann.

4. Medizinische Dichtungsvorrichtung (1) nach einem der Ansprüche 1-3, **dadurch gekennzeichnet, dass** die Dicke des Abdichtelements (5) weniger als 5 mm beträgt.

5. Medizinische Dichtungsvorrichtung (1) nach einem der vorhergehenden Ansprüche, **dadurch gekennzeichnet, dass** die medizinische Dichtungsvorrichtung (1) aus einem porösen Material besteht.

6. Medizinische Dichtungsvorrichtung (1) nach Anspruch 5, **dadurch gekennzeichnet, dass** die Porosität des Materials im Bereich von 50 bis 95 Prozent liegt.

7. Medizinische Dichtungsvorrichtung (1) nach einem der vorhergehenden Ansprüche, **dadurch gekennzeichnet, dass** das Abdichtelement (5) aus Glykolid, Laktid, Trimethylencarbonat, ε-Caprolakton, Dioxanon, Dioxepanon, Polyurethan, Polyphosphazenen, Polyorthoestern, Kollagen, Hyaluronsäure, Alginsäure, Fibrin, Stärke oder Hemizellulose oder Kombinationen davon besteht.

8. Medizinische Dichtungsvorrichtung (1) nach einem der vorhergehenden Ansprüche, **dadurch gekennzeichnet, dass** das Abdichtelement ferner ein hämostatisches Mittel umfasst.

9. Medizinische Dichtungsvorrichtung (1) nach Anspruch 8, **dadurch gekennzeichnet, dass** das hämostatische Mittel ausgewählt ist aus der Gruppe umfassen Thrombin, Prothrombin, Gerinnungsfaktor XIIa, Faktor Va, Faktor Xa oder Tranexamsäure.

## Revendications

1. Dispositif médical de fermeture (1) destiné à fermer une perforation dans la paroi d'un vaisseau, comprenant un élément intérieur (2) adapté pour être placé sur la surface intérieure due la paroi du vaisseau et un élément, extérieur (3) adapté pour être placé à l'extérieur de la paroi du vaisseau, l'élément intérieur (2) et l'autre élément (3) étant maintenus ensemble par an élément de retenue (4),
**caractérisé en ce que**
le dispositif de fermeture (1) comprend en outre un élément de fermeture (5) qui est une pièce séparée du dispositif de fermeture (1) et qui est adapté pour être placé entre la paroi du vaisseau et l'élément extérieur (3),
**en ce que** l'élément d'étanchéité est constitué d'un matériau malléable est déformable,
**en ce que** le diamètre de l'élément de fermeture (5) est essentiellement égal au diamètre de l'élément extérieur (3) est son épaisseur est comparable à l'épaisseur de l'élément intérieur et de l'élément: extérieur et
**en ce que** ledit élément de fermeture (5) est réalisé en un matériau biorésorbable.

2. Dispositif médical de fermeture (1) selon la revendication 1, **caractérisé en ce que** l'épaisseur de l'élément de fermeture (5) est plus grande dans sa partie centrale que dans sa partie périphérique.

3. Dispositif médical de fermeture (1) selon la revendication 2, **caractérisé en ce que** ladite partie centrale a une structure sen forme de cône qui peut pénétrer d'une courte distance dans ledit trou présent dans ladite paroi du vaisseau.

4. Dispositif médical de fermeture (1) selon l'une quelconque des revendications 1 à 3, **caractérisé en ce que** l'épaisseur de l'élément de fermeture (5) est inférieure à 5 mm.

5. Dispositif médical de fermeture (1) selon l'une quelconque des revendications précédentes, **caractérisé en ce que** le dispositif médical de fermeture (1) est réalisé en un matériau poreux.

6. Dispositif médical de fermeture (1) selon la revendication 5., **caractérisé en ce que** la porosité du matériau est comprise entre 50 et 95 pour cent.

7. Dispositif médical de fermeture (1) selon l'une quelconque des revendications précédentes, **caractérisé en ce que** l'élément de fermeture (5) est constitué de glycolide, de lactide, de carbonate de triméthylène, d'ε-caprolactone, de dioxanone, de dioxépanone, de polyuréthane , de polyphosphazènes, de polyorthoesters, de collagène, d'acide hyaluronique, d'acide alginique, de fibrine, d'amidon, d'hémicellulose ou de leurs combinaisons.

8. Dispositif médical de fermeture (1) selon l'une quelconque des revendications précédentes, **caractérisé en ce que** l'élément de fermeture comprend en outre un agent hémostatique.

9. Dispositif médical de fermeture (1) selon la revendication 8, **caractérisé en ce que** l'agent hémostatique est sélectionné dans l'ensemble qui comprend la thrombine, la pro-thrombine, le facteur de coagulation XIIa, le facteur Va, le facteur Xa ou l'acide tranexamique.
